Europäisches Patentamt

(19) European Patent Office

Office européen des brevets

(11) Numéro de publication: **0 204 877**
A1

# (12) DEMANDE DE BREVET EUROPEEN

(21) Numéro de dépôt: 85401134.3

(22) Date de dépôt: 10.06.85

(51) Int. Cl.⁴: **A 61 K 35/78**
A 61 K 7/48

(43) Date de publication de la demande:
17.12.86 Bulletin 86/51

(84) Etats contractants désignés:
AT BE CH DE FR GB IT LI LU NL SE

(71) Demandeur: **P.F. MEDICAMENT**
**125, Rue de la Faisanderie**
**F-75116 Paris(FR)**

(72) Inventeur: **Fauran, François**
**Bouisset-Labege**
**F-31320 Castanet Tolosan(FR)**

(72) Inventeur: **Couzinier, Jean-Pierre**
**Mandoul-Carbes**
**F-81100 Castres(FR)**

(72) Inventeur: **Hatinguais, Philippe**
**Le Landou-Chemin de Bel Air**
**F-81100 Castres(FR)**

(74) Mandataire: **Ahner, Francis et al,**
**CABINET REGIMBEAU 26, avenue Kléber**
**F-75116 Paris(FR)**

(54) Compositon dermatologique topique pour le traitement de l'acné, à base d'une fraction lipidique extraite de fruits de Serenoa repens.

(57) La présente invention concerne une composition dermatologique topique pour le traitement de l'acné, à base d'une fraction lipidique extraite de fruits de Serenoa repens.

La composition selon l'invention se caractérise en ce qu'elle contient à titre de principe actif 10 à 60 % en poids, et de préférence 20 à 50 % en poids, d'une fraction lipidique de fruits de Serenoa repens, ainsi qu'un support assurant la pénétration et la rémanence du principe actif au niveau de la peau.

EP 0 204 877 A1

COMPOSITION DERMATOLOGIQUE TOPIQUE POUR LE TRAITEMENT DE L'ACNE, A BASE D'UNE FRACTION LIPIDIQUE EXTRAITE DE FRUITS DE SERENOA REPENS.

La présente invention, réalisée au Centre de Recherches PIERRE FABRE, concerne de nouvelles compositions dermatologiques topiques à base d'une fraction lipidique extraite de fruits de Serenoa repens, qui sont destinées au traitement de l'acné.

Le Serenoa repens est un petit palmier des Etats-Unis, de 1 à 7 m de haut, abondant dans les états du Sud-Est des Etats-Unis, essentiellement entre l'embouchure du Mississipi et la Caroline du Nord. On le rencontre sur des terrains sablonneux arides et incultes.

La partie utilisée pour la réalisation de l'extrait est le fruit, drupe monosperme ovoïde de 1,5 à 2 cm de long et 1 à 1,5 cm de diamètre, de couleur noire rougeâtre.

La graine est lisse, ovoïde et mate, de 1 cm de long environ et de 0,4 à 0,6 cm de diamètre, à testa dur à embryon latéral dans un albumen homogène.

Les fruits sont récoltés à maturité d'août à Janvier suivant les régions.

Les extraits de Serenoa repens sont des produits connus pour leurs propriétés thérapeutiques exclusivement de type inhibiteur au niveau prostatique. Un tel extrait est d'ailleurs utilisé précisément pour ce type d'indications très ponctuelles, c'est-à-dire pour le traitement de troubles fonctionnels liés à l'hypertrophie bénigne de la prostate. Il convient, à ce propos, de préciser que ces extraits de Serenoa repens ont été administrés exclusivement par voie interne.

En revanche, la présente invention concerne une composition dermatologique destinée à l'administration topique pour le traitement de l'acné. Les compositions dermatologiques selon l'invention sont caractérisées en ce qu'elles contiennent à titre de principe actif 10 à 60 % en poids, de préférence 20 à 50 % en poids d'une fraction lipidique extraite à partir de Serenoa repens, ainsi qu'un support assurant la pénétration et la rémanence du principe actif au niveau de la peau.

Le principe actif présent dans les compositions selon l'invention peut être obtenu, par exemple en utilisant le mode opératoire suivant :

L'échantillon botanique utilisé est la drupe monosperme ovoïde de Serenoa repens.

Les fruits sont récoltés à maturité d'août à janvier suivant les régions.

L'extrait utilisé pour mettre en oeuvre la présente invention peut être préparé avantageusement, en utilisant comme solvant d'extraction un solvant apolaire tel que l'hexane en présence d'un antioxydant ajouté dès les premières phases de l'extraction. L'antioxydant est avantageusement choisi parmi le palmitate d'ascorbyle, les tocophénols, le gallate d'isopropyle. Les drupes sont broyées, dans un réacteur de 600 litres, 70 kg de cette poudre végétale, de granulométrie 0,5-1,5 mm. est mise en suspension dans 300 litres d'hexane contenant 5 g de palmitate d'ascorbyle. L'agitation est maintenue une heure en atmosphère d'azote, puis le marc est essoré et lavé par 50 litres d'hexane. Les solutions hexaniques réunies sont préconcentrées dans un appareil à évaporation continue de type LUWA, puis le solvant est fini d'évaporer dans un réacteur sous 3333 Pa à 6666 Pa, avec balayage d'azote en continu et chauffage à 60-70°C. On obtient ainsi une huile de couleur brunâtre qui est traitée par 2 % de charbon de type CECA 2 SA ou 3 SA, à 50° environ, sous atmosphère d'azote. Par filtration, on obtient une huile dépigmentée de couleur jaune orangé.

L'absence de solvant résiduel est contrôlée par chromatographie en phase vapeur. Le taux maximum est de 300 ppm.

Le produit ainsi obtenu possède les caractéristiques suivantes :

. caractères organoleptiques : liquide limpide
à 20°, huileux, de couleur jaune orangé,

. densité : voisine de 0,900,

. indice de réfraction : voisin de 1,452 (20°C),

. solubilités : soluble dans les solvants organiques et l'alcool, insoluble dans l'eau,

. indice d'iode : compris entre 45 et 55,

. indice de saponification : voisin de 230,

. insaponifiables : 1,8 à 3,5 g/ 100 g,

. identification des acides gras :
réalisée par CGL : colonne 4 % DEGS, sur chromosorb, 3 mètres
T. injecteur-détecteur 250°C
T. four 100 à 210°C (4°C/min.)
acides détectés : $C_6$-$C_8$-$C_{10}$-$C_{18}$-(0=)$C_{18}$(2=)
et $C_{20}$
et principalement : $C_{12}$-$C_{14}$-$C_{16}$ et $C_{18}$ (1=).

L'analyse du produit par CPG-SM met en évidence
du β-sitostérol, du stigmastérol et du campestérol, ainsi
que du cycloarténol, de la lupen-3 one, de l'hexacosanol,
de l'octacosanol et du triacontanol et des alcools de
haut poids moléculaire.

Il est avantageux d'utiliser, dans le cadre de
la présente invention, un extrait concentré en substances
actives, en soumettant l'extrait hexanique défini précédemment à une distillation fractionnée sous vide. Au cours
d'une telle phase de purification, les substances les plus
volatiles, liquides à la température ambiante, sont éliminées en tant que fraction de tête. La fraction de coeur,
correspondant à l'extrait concentré en principes actifs, est distillée à une température de 115°C à 135°C sous une pression d'environ 4 Pa.

Cette fraction de coeur représente 15 à 50 % de l'extrait hexanique mis en oeuvre, et possède les caractéristiques suivantes :

. caractères organoleptiques : solide à 20°C, gras au toucher, de couleur blanche, pratiquement inodore,

. point de fusion : 40 - 45°C ,

. solubilité : soluble dans les solvants apolaires et dans l'alcool ; insoluble dans l'eau.

. identification des acides gras : réalisée par CGL sur colonne 4 % DEGS sur chromosorb T. injecteur 250°C T. four 100°C à 210°C (4°C/min.)

Les acides gras de $C_{10}$ à $C_{20}$ sont détectés avec une majorité pour les groupes d'acides à $C_{12}$, $C_{14}$, $C_{16}$, et $C_{18}$, les acides en $C_{18}$ étant également mono ou diéthyléniques.

Il est bien entendu que cette purification, en plus de l'avantage d'un principe actif parfaitement compatible avec une utilisation dermo-cosmétologique par ses propriétés physicochimiques appropriées à une application topique, permet grâce à la concentration des principes actifs, une action encore plus efficace.

C'est de façon tout-à-fait inattendue que la demanderesse a constaté, par administration par voie topique chez l'animal de fractions lipidiques de fruits de Serenoa repens, soit sous forme d'extrait hexanique, soit sous forme de fraction active obtenue par distillation dans les conditions citées plus haut, une amélioration des troubles fonctionnels des glandes sébacées ou pilosébacées, et en particulier de l'acné expérimentale provoquée par dépôt de goudron.

A la suite de ces observations, diverses dispersions dosées de 10 à 60 % en poids d'extrait de Serenoa repens dans le mélange éthanol-eau, ont été expérimentées par voie locale.

Préalablement à cette expérimentation, la tolérance locale a été recherchée sur 5 cobayes albinos. L'application d'extrait hexanique de Serenoa repens non dilué, sur le flanc préalablement tondu, trois fois par semaine pendant 2 semaines, n'a provoqué aucune réaction cutanée appréciable.

La tolérance oculaire des extraits hexaniques de Serenoa repens a été réalisée sur des lapins albinos et sur des souris.

Après instillation dans le sac conjonctival de deux gouttes de solution aqueuse (concentrations de 10 à 60 % en poids en suspension aqueuse), on observe le comportement de l'animal pendant trois minutes. Pour toutes les concentrations testées, aucune différence n'a été observée avec les lots d'animaux témoins.

Des tests épicutanés ont été effectués avec des lapins de race néozélandaise, après avoir tondu les flancs on procède à des égratignures du stratum cornéum. Les résultats évalués d'après l'échelle de Draize ont montré que pour les plus faibles concentrations (inférieures à 60 %) l'indice d'irritation est très faible.

A la concentration de 60 %, une légère irritation apparaît, mais elle est limitée à un erythème léger.

Toutes ces observations effectuées montrent qu'il n'y a ni irritation primaire, ni phénomène de sensibilisation, ce qui a permis de tester le produit par voie locale chez l'homme.

La même série de tests de tolérance a confirmé une parfaite tolérance de la fraction purifiée par distillation.

### Clinique

Après étude de tolérance chez l'animal puis chez l'homme, quatre malades acnéiques ont été traités par application bi-quotidienne d'une lotion à l'extrait de Serenoa repens. Les résumés d'observations sont rapportés ci-après :

- Mlle G.R... 26 ans. Acné du visage. Intolérance au benzoyl peroxyde et à la trétinoïne. Amélioration évidente dès la 4ème semaine du traitement (pas de recrudescence prémenstruelle). Après deux mois, seule persiste une très ancienne formation enkystée de la joue.

- M. J.T... 24 ans. Acné banale du visage et du dos avec nombreuses formations nodulaires manifestement améliorée à la fin du 2ème mois de traitement.

- Mme A.C... 30 ans. Acné du visage apparue à l'adolescence très diminuée par un premier traitement de trois mois. Une récidive survient peu après le remplacement de la lotion par l'excipient. La reprise du traitement par la lotion est suivie d'un effet favorable.

- Mlle C.V... 14 ans. Le traitement évite totalement la recrudescence habituelle d'une acné du dos après le retour de vacances à la mer.

Le support utilisé dans les compositions dermatologiques topiques, objet de la présente invention, est choisi de manière à assurer la pénétration et la rémanence des extraits hexaniques de Serenoa repens au niveau de la peau. Un tel type de support peut par exemple être avantageusement constitué principalement par la combinaison de 20 à 70 % en volume d'alcool éthylique, le solde étant formé soit d'eau, soit de glycérol, soit d'un polyol tel que l'éthylèneglycol, le propylèneglycol ou leurs homologues oxyéthylénés pris seuls ou en mélanges. Peuvent être éga-

lement associées en petites quantités (inférieures à 10 % de la composition totale) des cétones telles que la méthylisobutylcétone, les esters d'alcool tel que l'acétate d'éthyle ou des éthers tels que le diméthoxyméthane, qui permettent de concourir à l'effet recherché sans causer d'effet désagréable au niveau de la peau.

Les compositions dermatologiques topiques, objet de la présente invention peuvent se présenter indifféremment sous la forme de solutions, de lotions, de gels ou de crèmes. Les solutions, gels ou lotions peuvent être conditionnés de façon classique sous la forme de flacons, d'aérosols ou encore d'ampoules.

Dans les compositions dermatologiques topiques, objet de la présente invention, l'extrait hexanique de Serenoa repens peut être associé à d'autres principes actifs par exemple choisis parmi les sels de zinc et notamment parmi le sulfate de zinc et le rétinoate de zinc.

Formulations

A titre d'illustration de l'invention, on indiquera ci-après quelques exemples non limitatifs de formule de compositions conformes à l'invention contenant comme principe actif un extrait hexanique de Serenoa repens seul ou associé à d'autres principes actifs.

Crème anti-acné :

- Fraction active distillée
  de Serenoa repens ................ 10 %
- Excipient .....................qsp 100 g

Crème anti-acné :

- Extrait hexanique de Serenoa repens    50 %
- Vitamine A...................... 0,1 %
- Conservateur
- Stéarate de polyéthylène glycol
- eau

0204877

<u>Gel dermique anti-acné</u> :

- Extrait hexanique de
  Serenoa repens ........... 30 %
- Rétinoate de Zn .......... 25 mg pour 100 g
- Alcool éthylique
- Klucel H.F.
- Conservateur.

<u>Composition pour traitement anti-acné</u> :

- Extrait de Serenoa repens . 10 %
- Rétinoate de Zn .......... 0,1 à 1 %
- Polyéthylène glycol
- Alcol éthylique

<u>Lotion anti-acné</u> :

- Fraction active distillée
  de Serenoa repens ........ 10 %
- Sulfate de zinc ......... 1 %
- Acide salicylique ........ 1 %
- colorant - parfum -
  conservateur ..........qs
- eau

## REVENDICATIONS

1. - Composition dermatologique topique pour le traitement de l'acné, caractérisée en ce qu'elle contient à titre de principe actif 10 à 60 % en poids, et de préférence 20 à 50 % en poids, d'une fraction lipidique de fruits de Serenoa repens, ainsi qu'un support assurant la pénétration et la rémanence du principe actif au niveau de la peau.

2. - Composition selon la revendication 1, caractérisée en ce que la fraction lipidique de Serenoa repens est une fraction enrichie par distillation sous vide d'un extrait hexanique de fruits de Serenoa repens, ladite fraction enrichie ayant une teneur prépondérante en acides en $C_{12}$, $C_{14}$, $C_{16}$ et $C_{18}$.

3. - Composition selon l'une des revendications 1 et 2, caractérisée en ce qu'elle contient de 20 à 50 % en poids d'une fraction lipidique de fruits de Serenoa repens en association avec d'autres principes actifs tels que les sels de zinc et notamment de rétinoate de zinc.

4. - Composition selon l'une des revendications 1 à 3, caractérisée en ce qu'elle se présente sous la forme de solutions, de lotions, de gels ou de crèmes.

Office européen
des brevets

## RAPPORT DE RECHERCHE EUROPEENNE

EP 85 40 1134

| | DOCUMENTS CONSIDERES COMME PERTINENTS | | |
|---|---|---|---|
| Catégorie | Citation du document avec indication, en cas de besoin, des parties pertinentes | Revendication concernée | CLASSEMENT DE LA DEMANDE (Int. Cl.4) |
| E | FR-A-2 556 968 (P.F. MEDICAMENT) <br> * Page 9, revendications 1-4 * | 1-4 | A 61 K 35/78 <br> A 61 K 7/48 |
| X | EXPERIENTIA, vol. 25, no. 8, 15 auoût 1969, pages 828,829, Bâle, CH; M.I. ELGHARMRY et al.: "Activity and isolated phytoestrogen of shrub palmetto fruits (Serenoa repens small), a new estrogenic plant" <br> * Page 829, résumé * | 1-4 | |
| X | EP-A-0 068 055 (P. FABRE SA) <br> * Page 5, revendications 1-9 * | 1-4 | |
| X | FR-A-2 480 754 (P. FABRE SA) <br> * Page 4, revendications 1-9 * | 1-4 | DOMAINES TECHNIQUES RECHERCHES (Int. Cl.4) <br><br> A 61 K |

Le présent rapport de recherche a été établi pour toutes les revendications

| Lieu de la recherche <br> LA HAYE | Date d'achèvement de la recherche <br> 10-02-1986 | Examinateur <br> BRINKMANN C. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03 82